# EUROPEAN PATENT APPLICATION

(11) **EP 3 020 916 A1**
(43) Date of publication of application: **18.05.2016**
(21) Application number: 14290346.7
(22) Date of filing: 14.11.2014
(51) Int. Cl.: E21B 49/00, G01N 33/24, G01N 33/28

(54) **A method for processing data collected during a mud logging analysis, associated calculation system and associated mud logging installation**

(71) Applicant: Geoservices Equipements, 95971 Roissy en France (FR)
(72) Inventor: Guerriero, Nicolas, 75011 Paris (FR); Colombel, Emilie, 67049 Paris Nord II (FR); Dercq, Michaël, 75018 Paris (FR)
(74) Representative: Leonori, Céline

(57) **Abstract**

The disclosure relates to a method for processing data collected during a mud logging analysis, comprising:
- providing, at a plurality of measuring times, a set of gas measurement values representative of the quantity of at least one gas contained in several samples of a drilling fluid,
- providing, at a plurality of controlling times, a set of control values of a control parameter relative to part of an analysis unit for providing the gas samples or the gas measurement values;
- matching, for each sample of the drilling fluid, the gas measurement value measured at a given measuring time with at least one control value measured at a controlling time;
- evaluating whether each control value of the control parameter is in a normal state.

## Description

The present disclosure concerns a method for processing data collected during a mud logging analysis, comprising providing, at a plurality of measuring times, a set of gas measurement values representative of the quantity of at least one gas contained in several samples of a drilling fluid or mud.

### BACKGROUND

When drilling an oil well or a well for another effluent (in particular gas or water), it is known to carry out an analysis of the gaseous compounds contained in the drilling fluid or mud emerging from the well. This analysis makes it possible plays a part in determining the possibilities for exploiting the deposits of fluids encountered.

This analysis called "mud gas logging" or "fluid logging", which is carried out continuously, comprises three main phases. The first phase comprises extracting the gases carried by the mud (for example hydrocarbons, carbon dioxide, hydrogen sulphide, helium and nitrogen). The second phase comprises qualifying and quantifying the gases extracted. The third phase comprises determining the depths of the geological layers containing the extracted gas.

In the first phase, mechanically agitated degassers such as the ones disclosed in application FR-A-2 799 790 hereby incorporated by reference in its entirety are frequently used. The gases extracted from the mud, which are mixed with a carrier gas introduced into the enclosure, are conveyed by suction via the gas extraction pipe to an analyzer which permits the extracted gases to be quantified. Then, a data processing, such as the one performed in application US 2007/0062272, is carried out to determine the depths of the corresponding geological layers.

However, during the first and the second phases, experimental disturbances may lead to erroneous gas measurements or artefacts which make difficult the data processing of the third phase, leading to an analysis that may not provide accurate results.

It is therefore common that an operator manually filters the gas measurements which he considers erroneous. Nevertheless, such a filtering is time consuming and dependent on the operator who may filter good data and/or miss to filter some erroneous data.

### SUMMARY OF THE DISCLOSURE

The present disclosure provides a method for accurately determining erroneous gas measurement during a mud logging analysis.

The present disclosure relates to a method comprising:
- providing, at a plurality of measuring times, a set of gas measurement values representative of the quantity of at least one gas contained in several samples of a drilling fluid,
- providing, at a plurality of controlling times, a set of control values of a control parameter relative to part of an analysis unit for providing the gas samples or the gas measurement values;
- matching, for each sample of the drilling fluid, the gas measurement value measured at a given measuring time with at least one control value measured at a controlling time so that the control values are representative of the part of the analysis unit, when the sample was processed in said part of the analysis unit;
- evaluating whether each control value of the control parameter is in a normal state by comparing the control value with a predetermined value specific to the control parameter, in order to assign, to each control value, an indicator of the quality of the measurement of the gas measurement value.
   The method according to the disclosure may comprise one or more of the following characteristics taken in isolation or in any technically possible combinations,
- filtering out a gas measurement value when the indicator assigned to one of the matched control value indicates that the matched control value is not in a normal state;
- after filtering, calculating the depth at which each sample of the drilling fluid was extracted in order to assign a depth to each gas measurement value measured at a measuring time;
- the evaluation includes evaluating whether the control value is comprised in a first range of values specific to the control parameter;
- the evaluation comprises determining whether each control value of the control parameter is included in a second range of values, the first range of values being included in the second predetermined range of values, the indicator assigned to each control value being of a first type when the control value is comprised in the first range of values, of a second type when the control value is comprised in the second range of values and of a third type when the control value is included neither in the first nor in the second range of values. Of course, more ranges and corresponding indicators may also be set up ;
- assessing, for each control parameter, a time difference between, the measurement of a gas measurement value of a sample of the drilling fluid and the measurement of a control value of each control parameter associated to the same sample of the drilling fluid, by experiment or/and calculation. Matching may also comprise: adding to the controlling times of the control values of each control parameter, the assessed time difference corresponding to the control parameter, to obtain an adjusted time for each control value, and affecting each control value to the gas measurement value whose measuring time is the closest to the adjusted time of the control value;
- the evaluation comprises validating and /or modifying each indicator affected to each control value manually;
- providing the gas measurement value comprises:
- introducing a sample of a drilling fluid in an extractor of the analysis unit;
- extracting a gas stream containing the gas to be measured out of the extractor; and
- introducing at least part of the gas stream in an analyzer of the analysis unit to obtain a gas measurement value;
- providing the control values comprises measuring a control parameter chosen among the following group: a mud level in the extractor, a flow rate of mud introduced in the extractor, a temperature of the gas stream inside the extractor, a pressure of the gas stream after being extracted and filtered, before entering the analyzer, a flow rate of the gas stream in a pump and a pressure of the gas stream in a pump.

The disclosure also relates to a calculation system comprising a processing unit in interaction with a software application for the implementation of the method as mentioned above.

The disclosure also relates to a mud logging installation comprising:
- an analysis unit for providing gas samples or gas measurement values representative of the quantity of at least one gas contained in several samples of a drilling fluid extracted from a well bore, comprising :
   - a sampling unit for sampling the drilling fluid,
   - an extractor able to extract the gases contained in the samples of the drilling fluid,
   - an analyzer able to measure the quantity of at least one gas contained in the samples of the drilling fluid, and
   - sensors able to measure at least one control parameter of the analysis unit for providing gas samples and/or gas measurement values, and
- a calculation system as mentioned above.

### BRIEF DESCRIPTION OF THE DRAWINGS

The disclosure will be better understood on reading the following description, which is given solely by way of example, and is written with reference to the appended drawings, in which:
- Figure 1 is a schematic view in vertical section of a drilling rig comprising a mud logging installation according to an embodiment of the disclosure,
- Figure 2 is an organization chart of a method according to the an embodiment of the disclosure, and
- Figure 3 is a screenshot on the display unit of a calculation system, representing a set of gas measurement values and a set of control values.

### DETAILED DESCRIPTION

One or more specific embodiments of the present disclosure will be described below. These described embodiments are examples of the presently disclosed techniques. Additionally, in an effort to provide a concise description of these embodiments, some features of an actual implementation may not be described in the specification. It should be appreciated that in the development of any such actual implementation, as in any engineering or design project, numerous implementation-specific decisions may be made to achieve the developers' specific goals, such as compliance with system-related and business-related constraints, which may vary from one implementation to another. Moreover, it should be appreciated that such a development effort might be complex and time consuming, but would still be a routine undertaking of design, fabrication, and manufacture for those of ordinary skill having the benefit of this disclosure.

When introducing elements of various embodiments of the present disclosure, the articles "a," "an," and "the" are intended to mean that there are one or more of the elements. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features.

In the following description, the terms "upstream" and "downstream" are understood with respect to the normal direction of circulation of a fluid in a pipe.

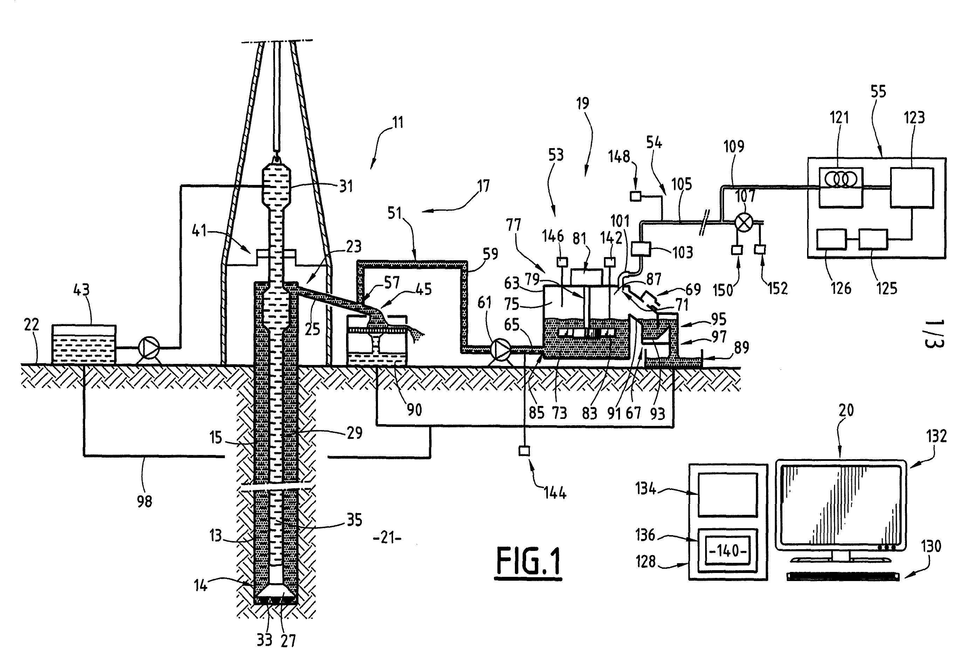

A mud logging installation 11 is illustrated on Figure 1.

This mud logging installation 11 comprises a drilling pipe 13 arranged in a cavity 14 bored by a rotary drilling tool 15, a surface installation 17, an analysis unit 19 able to measure at least a gas measurement value V₁ representative of the gas contained in the mud and a calculation system 20.

The drilling pipe 13 is arranged in the cavity 14 formed in the earth formation 21 by the rotary drilling tool 15. This pipe 13 comprises, at the surface 22, a well head 23 provided with a discharge pipe 25.

The drilling tool 15 comprises a drilling head 27, a drill string 29 and a liquid injection head 31.

The drilling head 27 comprises a drill bit 33 for drilling through the rocks of the earth formation 21. It is mounted on the lower portion of the drill string 29 and is positioned in the bottom of the drilling pipe 13.

The string 29 comprises a set of hollow drilling tubes. These tubes delimit an inner space 35 which makes it possible to bring a liquid from the surface 22 to the drilling head 27. To this end, the liquid injection head 31 is screwed onto the upper portion of the string 29.

The surface installation 17 comprises a rotator 41 for supporting the drilling tool 15 and driving it in rotation, an injector 43 for injecting the drilling liquid and a shale shaker 45.

The injector 43 is hydraulically connected to the injection head 31 in order to introduce and circulate a liquid in the inner space 35 of the drill string 29.

The shale shaker 45 collects the liquid laden with drilling residues which emerges from the discharge pipe 25, and separates the liquid from the solid drilling residues.

The analysis unit 19 is an installation for providing gas samples and gas measurement values from the gas samples. The analysis unit 19 comprises a sampling unit 51 for sampling the drilling fluid or mud which is tapped into the discharge pipe 25, a gas extractor 53 and a line 54 for transporting the extracted gases which is connected to the extractor 53. The analysis unit 19 further comprises an analyzer 55 for the extracted gases, downstream from the extractor 53, into which the transport line 54 opens.

The sampling unit 51 comprise a liquid sampling head 57, arranged protruding into the discharge pipe 25, a connecting tube 59 and a peristaltic pump 61, the flow rate of which is adjustable.

In a variant, the sampling unit 51 is tapped into a receiving tank for the liquid, into which the discharge pipe 25 opens. In another variant, the sampling unit 51 is tapped into a tank of the mud injector 43.

The extractor 53 comprises an enclosure 63, a mud supply pipe 65 for supplying mud into the enclosure 63, an evacuation pipe 67 for evacuating the mud from the enclosure 63, an inlet 69 for introducing a carrier gas into the enclosure 63 and an outlet 71 for extracting the extracted gases from the enclosure 63.

The enclosure 63 comprises a hermetic receptacle, the internal volume of which is for example between 0.4 litres and 3 litres. This enclosure 63 comprises a lower portion 73, in which the mud circulates, and an upper portion 75. The enclosure 63 is further provided with an agitating unit 77, comprising an agitator 79, mounted so as to protrude into the enclosure 63 and driven in rotation by a motor 81 mounted on the upper portion 75 of the enclosure 63. The agitator 79 comprises an agitating mechanism 83 immersed in the mud.

The mud supply pipe 65 extends between the outlet of the peristaltic pump 61 and an entry opening 85, formed in the lower portion 73 or upper portion 75 of the enclosure 63.

This supply pipe 65 may be provided with a heater for heating the mud (not shown) in order to bring the temperature of the mud to values of between 25 and 150 °C, e.g. of between 60 and 90 °C.

The evacuation pipe 67 extends between an overflow passage 87, formed in the upper portion 75 of the enclosure 63, and a retention basin 89 for receiving the mud evacuated from the device 53.

In a variant, the retention basin 89 is formed by a receiving tank 90 for the liquids extracted from the shale shaker 45.

The evacuation pipe 67 here comprises in succession an upstream portion 91 inclined downwards, which is at an angle of approximately 45° to the horizontal, an angled portion 93 forming a siphon, and a substantially vertical downstream portion 95, open at its lower end 97 arranged facing the basin 89, above the level of the liquid contained in the basin 89.

The mud collected in the retention basin 89 and in the tank 90 is recycled to the injector 43 by a mud recirculation pipe 98.

The introduction inlet 69 opens into the upper portion 75 of the enclosure 63. It is connected to a source (not shown) of an inert carrier gas such as nitrogen or helium. In a variant, the inlet 69 opens into the atmosphere located around the enclosure 63. The carrier gas is chosen for not reacting with the gas extracted from the wellbore and that will be analysed afterwards.

The outlet for evacuating the extracted gases 71 is delimited in an upper portion of the enclosure 63, in the vicinity of the agitator 79. It comprises a fitting 101 for connection to the transport line 54.

The line 54 is mounted on the fitting 101. The line 54 is capable of continuously sampling a stream of gases extracted from the mud in the upper portion 75 of the enclosure 63 in order to convey this stream to the analyzer 55.

As will be seen below, this gas stream contains hydrocarbons to be analysed, water vapour and other inorganic compounds. The hydrocarbons to be analysed are for example C₁ to Cₙ hydrocarbons, with n being less than or equal to 10, for instance

The transport line 54 connects the enclosure 63 arranged in the vicinity of the well head 23, in the explosive zone, to the analyzer 55, which is arranged spaced apart from the well head 23, in a non-explosive zone, for example in a pressurised cabin. In a variant, the line 54 is very short and the analyzer 55 is placed in the vicinity of the well head.

The transport line 54 may be produced on the basis of a material which is inert towards the gaseous compounds extracted from the mud, such as steel, polyethylene (PE) or PTFE. It has for example a length which varies between 10 cm and 500 m.

The transport line 54 is provided, from upstream to downstream, with a filtration module 103, a flow rate controller 105 located in the vicinity of the enclosure 63, a vacuum pump 107 for conveying the extracted gases, and a branch connection 109 for connection to the analyzer 55 opening upstream from the pump 107.

The filtration module 103 is capable of creating a pressure drop leading to a partial vacuum. For example, this filtration module 103 comprises a water trap, a particle filter and a flow restrictor to lead to a partial vacuum.

The water trap comprises at least one cold water condensation surface in order to eliminate the water vapour present in the extracted gases substantially by condensation.

The particle filter is, for example, a demister filter.

The flow rate controller 105 is formed by a tube having a constriction of calibrated cross-section. The controller sets a volume flow rate for the flow of extracted gases which circulates in the line 54. This flow rate is for example of between 300 cm³ per minute and 2000 cm³ per minute, and for instance equal to 500 cm³ per minute.

The pump 107 permits conveying by suction of the gases extracted from the enclosure 63 to the analyzer 55. It is placed in the vicinity of the analyzer 55. It has an inlet connected to the line 54 in parallel to the branch connection 109 and an evacuation outlet which opens into the atmosphere.

The branch connection 109 opens upstream from the inlet into the pump 107. It is capable of sampling a predetermined percentage (approximately 10 %) of the volume flow rate of extracted gases circulating in the line 54, the rest of the flow of extracted gases circulating through the pump 107 to be evacuated into the atmosphere.

The analyzer 55 comprises a separation column 121 for the gases to be analysed, a detector 123 for successive detection of the gases which are separated in the separation column 121, a qualification and/or quantification unit 125 for qualification and/or quantification of the gases to be analysed which are detected by the detector 123 and a memory 126.

The separation column 121 may be a gas chromatograph, for instance a gas-chromatography separation column. This column is for example charged with the aid of a stationary phase in the form of a gel which permits the selective dissolution of the gases in the gel in order to retain them selectively (gas-liquid chromatography). In a variant, the column has a solid coating capable of interacting with the gases to be analysed in order to retain them selectively according to their affinity with the coating (gas-solid chromatography).

The separation column is capable of eluting in succession the gases to be analysed according to the number of atoms which they comprise, starting from a stream injected at the inlet containing the gases to be analysed at a given moment. The gases to be analysed emerge from the column 121 at distinct elution times of between 10 s and 100 s.

The detector 123 is for example a flame ionisation detector (FID), and/or a thermal conductivity detector (TCD). The detector may possibly be a mass spectrograph, depending on the analysis required on the gases.

The qualification and/or quantification unit 125 is capable of qualifying the gases. In particular, the qualification and/or quantification unit 125 is capable of qualifying the C₁ to Cₙ hydrocarbons with n being less than or equal to 10, more specifically with n being less than or equal to 8, in order to detect their presence in the gas stream, and of quantifying the relative contents of at least the C₁ to C₆ hydrocarbons.

The memory 126 is capable of storing data coming from the qualification and/or quantification unit 125.

The calculation system 20 is, for example, a computer.

The calculation system 20 comprises a processor 128, a man-machine interface 130 and a display unit 132.

The processor 128 comprises a processing unit 134, a memory 136 and a software application 140 stored in the memory 136. The software application 140 is configured to be executed by the processing unit 134.

The man-machine interface 130 is, for example, a touchscreen or a keyboard.

The display unit 132 is, for example, a computer screen.

The analysis unit 19 also includes sensors for measuring at least one control parameter of the unit 19. Each of the control parameters is relative to a part of the unit 19 for providing the gas samples, for instance relative to the sampling and transport of the mud or to the gas extraction, and/or for providing the gas measurements, for instance relative to the transport of the gas stream obtained from the samples of the drilling fluid.

The at least one control parameter is chosen among: a mud level in the extractor 53, a flow rate of mud introduced in the extractor 53, a temperature of the gas stream inside the extractor 53, a pressure of the gas stream after being extracted and filtered, before entering the analyzer 55, a flow rate of the gas stream in the vacuum pump 107 and a pressure of the gas stream in the vacuum pump 107. However, it is also possible to choose other control parameters.

For instance on figure 1, six sensors are represented: a mud level sensor 142 located inside the extractor 53, a mud flow rate sensor 144 located before the extractor 53 at the entry opening 85, a gas stream temperature sensor 146 located inside the extractor 53, a gas stream pressure sensor 148 located after the extractor 53 and the filtration module 103 and before the analyzer 55, a gas stream flow rate sensor 150 located at the vacuum pump 107 and a gas stream pressure sensor 152 located at the vacuum pump 107.

However, it is possible to have less or more sensors and/or sensors of different type and/or at different locations than those represented on figure 1.

The mud level sensor 142 is a sensor capable of measuring the presence of a liquid and/or a liquid level, in particular a mud level. The mud level sensor 142 is, for example, a Boolean sensor comprising two vibrating blades indicating if air or a liquid is in between.

The mud flow rate sensor 144 is a flow meter. It is understood by "flow meter", a device for measuring the flow of a fluid or of a gas. More precisely, the mud flow rate sensor 144 is a Coriolis flow meter providing a volume flow rate based on the temperature and the specific gravity of the fluid.

The gas stream temperature sensor 146 is a thermometer. More precisely, the gas stream temperature sensor 146 is a resistance thermometer such as a Pt 100 sensor.

The gas stream pressure sensor 148 is a manometer. More precisely, the gas stream pressure sensor 148 is a 4-20 mA manometer.

The gas stream flow rate sensor 150 is a flow meter. More precisely, the gas stream flow rate sensor 150 is a mass flow meter for gas.

The gas stream pressure sensor 152 is a manometer. More precisely, the gas stream pressure sensor 152 is a 4-20 mA manometer.

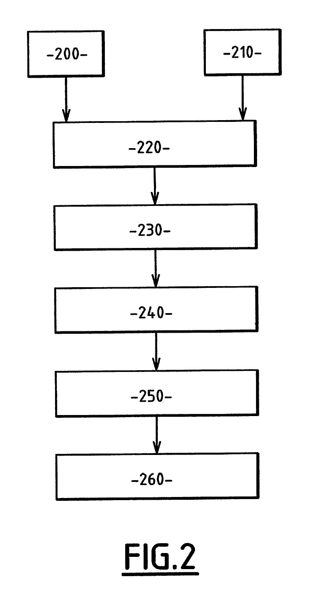

The method for processing data collected during a mud logging analysis, according to an embodiment of the disclosure will now be described, as an example, with reference to Figure 2.

Initially, the method comprises providing (box 100), at a plurality of measuring times t₁, a set of gas measurement values V₁. In other words, there is a gas measurement value V₁ for each measuring time t₁. Each gas measurement value V₁ is representative of the quantity of at least one gas contained in a sample of a drilling fluid. The at least one gas is for example chosen among: the C₁ to C₈ hydrocarbons, the C₄ to C₅ hydrocarbons branched isomers, aromatic species such as benzene and inorganic compounds such as CO₂, He, H, H₂S.

The gas measurement values V₁ are obtained during the drilling of a well in the ground. During this operation, the drilling tool 15 is driven in rotation by the rotator 41. A drilling liquid is introduced into the inner space 35 of the drill string 29 by the injector 43. This liquid moves downwards as far as the drilling head 27, and passes into the drilling pipe 13 through the drilling head 27. This liquid cools and lubricates the drill bit 33. Then the liquid collects the solid cuttings resulting from the drilling operation and moves back upwards through the annular space defined between the drill string 29 and the walls of the drilling pipe 13. Thereafter, the liquid is evacuated through the discharge pipe 25. The liquid containing the cuttings then forms the drilling fluid to be analysed.

The peristaltic pump 61 is then activated in order to sample continuously a given fraction of the drilling fluid circulating in the discharge pipe 25. Several mud samples or mud fractions of the drilling fluid can thus be obtained continuously.

Each mud fraction is conveyed to the enclosure 63 via the supply pipe 65, and is introduced into the enclosure 63.

The mud introduced into the enclosure 63 via the supply pipe 65 is evacuated by overflowing into the evacuation pipe 67 through the overflow passage 87. Furthermore, a portion of the evacuated mud temporarily resides in the siphon 93 of the evacuation pipe 67, which prevents gas from entering the upper portion 75 of the enclosure 63 through the lower end 97 of the evacuation pipe 67. The introduction of gas into the enclosure 63 therefore takes place solely through the introduction inlet 69.

The agitator 79 is driven in rotation by the motor 81, and agitates the mud in the lower portion 73 of the enclosure 63 in order to bring about the continuous extraction of the gases contained in the mud, and also the mixing of the extracted gases with the carrier gas introduced through the injection passage 99.

A stream of extracted gas is sampled continuously at the outlet 101 under the action of the suction caused by the pump 107. The stream of extracted gases comprises, for example, hydrocarbons, helium, nitrogen, hydrogen sulphide, carbon dioxide, water vapour.

The gas stream is then conveyed through the filtration module 103 in order to eliminate the particles and the water vapour present by condensation. The filtration module 103 also creates a pressure drop leading to a partial vacuum. The gas stream then flows through the flow rate controller 105. The controlled flow rate of gas stream circulating in the line 54 is then between 300 cm³/min and 2000 cm³/min.

Then, approximately 10 % of the gas stream is sampled through the branch connection 109, whereas approximately 90 % of the gas stream is transported to the atmosphere through the pump.

The gas stream present in the branch connection 109 is then introduced into the separation column 121 of the analyzer 55, which permits the selective separation of the gases according to their elution time in the column 121.

The successive presence and the quantity of these gases are detected through the detector 123. In particular, the detector 123 detects, at different measuring time t₁, a set of gas measurement values V₁ of the quantity of at least one gas contained in different samples of the drilling fluid. The detected data are then stored in the memory 126, each associated with a measuring time t₁.

The data contained in the memory 126 are then transferred to the memory 136 of the calculation system 20. The data transfer is, for example, carried out by a wireless transmission protocol, for instance of the type Wi-Fi or by a support such as a USB key, an optical disk, a CD-ROM, or a hard disk.

Thereafter, the method comprises providing (box 210), at a plurality of controlling times t₂, a set of control values V₂. In other words, there is a control value V₂ for each controlling time t₂. The control values V₂ are representative of the values of at least one control parameter monitoring at least part of the analysis unit 19 when providing the samples and/or the gas measurement values V₁.

The control values V₂ taken at controlling time t₂ may be associated with a sample and/or a set of gas measurement V₁. Indeed, each gas measurement value V₁, obtained from a sample of the drilling fluid, corresponds to at least one control value V₂ representative of part of the unit 19 while the sample (e.g. contained in the drilling fluid or in the gas stream) was processed in this part of the unit 19.

The controlling time t₂ and the measuring time t₁ corresponding respectively to a control value V₂ of a sample of the drilling fluid and to a gas measurement value V₁ of the same sample of the drilling fluid or of the gas stream obtained from this sample, may be shifted in time.

For example, the calculation system 20 is provided (box 210) with the control values V₂ of six control parameters: the mud level in the extractor 53, the flow rate of mud introduced in the extractor 53, the temperature of the gas stream inside the extractor 53, the pressure of the gas stream after being extracted and filtered, before entering the analyzer 55, the flow rate of the gas stream in the vacuum pump 107 and the pressure of the gas stream in the vacuum pump 107.

The providing 210 is carried out by at least one sensor, such as the sensors 142, 144, 146, 148, 150, 152 represented on figure 1.

Then, the obtained control values V₂ and their controlling times t₂ are transmitted to the memory 136 of the calculation system 20. The data transfer is, for example, carried out by a wireless transmission protocol of the type Wi-Fi or by a support such as a USB key, an optical disk, a CD-ROM, or a hard disk.

The providing 210 is conducted in parallel to the initial providing 200.

Then, the method comprises assessing (box 220) the time difference Δt between the measurement of a gas measurement value V₁, corresponding to a sample of the drilling fluid, and the measurement of the control value V₂ of each control parameter for the same sample of the drilling fluid.

For a given control parameter, the time differences Δt between the measurement of each gas measurement value V₁ corresponding to a sample of the drilling fluid and the measurement of each control value V₂ of the control parameter for the same sample of the drilling fluid, are considered constant. In other word, each control parameter has a single time difference Δt.

The time differences Δt are assessed by experiment and/or by calculation. For example, once all the operating parameters have been set properly and controlled, time difference measurements are performed by measuring, for instance, the time for water pumped to flow out of the extractor 53 or the time for gas from a gas cylinder connected at the air inlet 69 of the extractor 53 to reach the analyzer 55 (first peak observed on chromatograms). In another example, the volume of the extractor is calculated and time difference is then calculated knowing the mud flow rate.

The measuring times t₁ and the corresponding controlling times t₂ may be the same if the control parameter relates to the analyzer 55 while the gas measurement is being taken. The time differences Δt are then equal to zero.

Then, the method comprises a matching (box 230), for each sample of the drilling fluid, the corresponding gas measurement value V₁ measured at a given measuring time t₁ with at least one control value V₂ of the same sample of the drilling fluid measured at a corresponding controlling time t₂. In other words, if there is more than one control parameter, each gas measurement value V₁ is matched with the same number of control / value V₂ than the number of control parameters.

The matching 230 comprises adding to the controlling time t₂ of each control value V₂ of the same control parameter, the assessed time difference Δt corresponding to the control parameter. The times obtained are called "adjusted times tₐ". There is one adjusted time tₐ for each controlling time t₂.

The matching 230 further comprises affecting each control value V₂ to the gas measurement value V₁ whose measuring time t₁ is the closest to the adjusted time tₐ of the control value V₂.

Then, the method comprises evaluating (box 240) whether each control value V₂ of each control parameter corresponds to a normal state by comparing the control value V₂ with at least a predetermined value, specific to the control parameter. An indicator of the quality of the measurement of each gas measurement value V₁ matched with the control value V₂ is, then, assigned to each control value V₂.

The control value V₂ may also be in a normal state if it corresponds to a predetermined value, like a value of a Boolean sensor. For example, when the control parameter is the mud level in the extractor 53, the control value is in a normal state when it corresponds to a predetermined value (like the number 1 or TRUE) indicating that the sensor is immersed in the mud.

The control value V₂ may also be in a normal state if the control value V₂ is comprised in a first predetermined range of values R₁ of the control parameter corresponding to a normal value of the control parameter, and in an abnormal state if it is not in this range of values R₁. It may be in a second predetermined range of values R₂ of the control parameter which corresponds to a suspicious value of the control parameter or neither in the first nor in the second predetermined range of values R₁, R₂ corresponding to an abnormal value of the control parameter. The control value V₂ may also be in a normal state if it corresponds to a predetermined value, like a value of a Boolean sensor.

The first predetermined range of values R₁ and the second predetermined range of values R₂ may be characteristic of a control parameter. In other words there is the same number of first and second predetermined range of values R₂ than the number of control parameters.

The first predetermined range of values R₁ is included in the second predetermined range of values R₂.

For example, when the control parameter is the flow rate of mud introduced in the extractor 53, the first predetermined range of values R₁ is comprised between 275 cubic centimeters per minute (cc/min) and 325 cc/min, the second predetermined range of values R₂ is comprised between 250 cc/min and 350 cc/min.

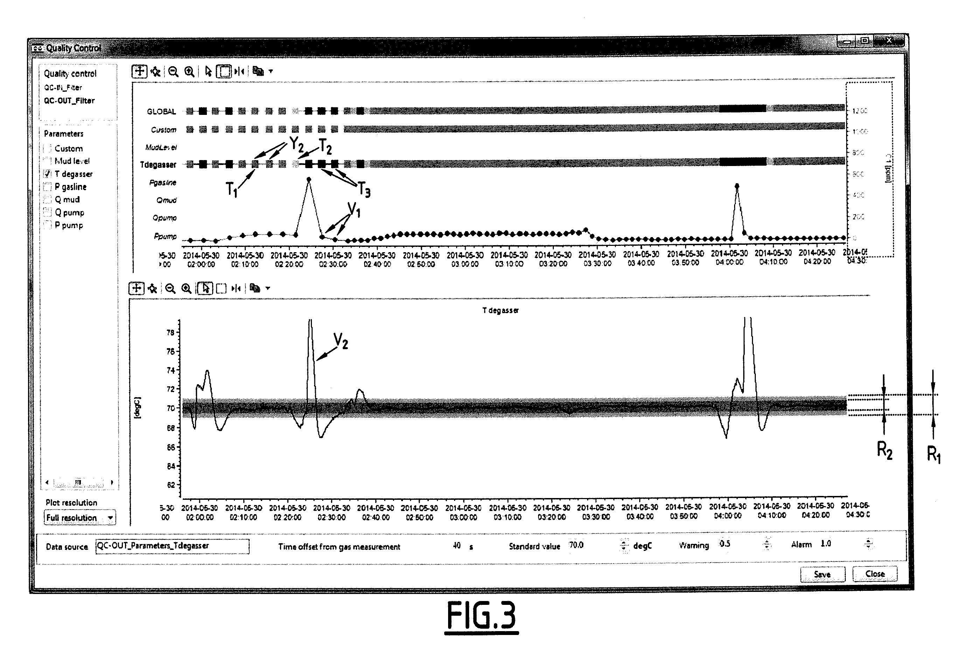

For example, as illustrated on figure 3, when the control parameter is the temperature of the gas stream inside the extractor 53, the first predetermined range of values R₁ is comprised between 69.5°C and 70.5 °C for water-based mud, the second predetermined range of values R₂ is comprised between 69°C and 71 °C for water-based mud. For oil-based mud, the first predetermined range of values R₁ is comprised between 89.5°C and 90.5 °C and the second predetermined range of values R₂ is comprised between 89°C and 91 °C.

For example, when the control parameter is the pressure of the gas stream after being extracted and filtered, before entering the analyzer 55, the first predetermined range of values R₁ is comprised between 250 millibars (mbar) and 270 mbar, the second predetermined range of values R₂ is comprised between 240 mbar and 280 mbar

For example, when the control parameter is the flow rate of the gas stream in the vacuum pump 107, the first predetermined range of values R₁ is comprised between 475 cc/min and 525 cc/min, the second predetermined range of values R₂ is comprised between 450 cc/min and 550 cc/min

For example, when the control parameter is the pressure of the gas stream in the vacuum pump 107, the first predetermined range of values R₁ 0 mbar and 100 mbar.

The evaluation 240 further comprises the assignment of an indicator to each control value V₂. There are three types of indicators: a first type T₁ of indicator when the control value V₂ is comprised in the first predetermined range of values R₁, a second type T₂ of indicator when the control value V₂ is comprised in the second predetermined range of values R₂ and a third type T₃ of indicator when the control value V2 is comprised in neither in the first nor in the second predetermined range of values R₁, R₂.

When a first type T₁ of indicator is affected to a control value V₂, this control value V₂ is considered acceptable.

When a second type T₂ of indicator is affected to a control value V₂, this control value V₂ is considered suspicious.

When a third indicator T₃ is affected to a control value V₂, this control value V₂ is considered highly suspicious and might be erroneous.

It is also possible to have more or less types of indicators and/or more or less predetermined range of values for each control parameter.

The gas measurement values V₁, the control values V₂ and their corresponding indicators are then displayed on the display unit 132.

The indicators are, for example, represented by colors applied on the representation of each control value V₂ on the display unit 132: a green color when the control value V₂ is comprised in the first predetermined range of values R₁, an orange color when the control value V₂ is comprised in the second predetermined range of values R₂ and a red color when the control value V₂ is comprised neither in the first nor in the second predetermined range of values R₁, R₂.

For example, as illustrated on the bottom of figure 3, the display unit 132 displays a first graph representing the control values V₂ of the temperature of the gas stream inside the extractor 53 as a function of the adjusted times tₐ. On this first graph, the first predetermined range of values R₁ is illustrated by a band of dark gray color and the second predetermined range of values R₂ is illustrated by two band of light gray color.

For example, as illustrated on the top of figure 3, the display unit 132 displays a second graph representing the gas measurement value V₁ of the quantity of a hydrocarbon C₁ as a function of the measuring times t₁. In particular, each gas measurement value V₁ is represented by a circle on a curve. On this second graph, the control values V₂ of the temperature of the gas stream inside the extractor 53 are represented by squares on a line whose abscissa is the measuring times t₁ of the gas measurement value V₁. The colors of the squares representing the control values V₂ corresponds to the type of indicators affected to each control value V₂. In particular, the squares have a light gray color when the control value V₂ is comprised in the first predetermined range of values R₁, a dark gray color when the control value V₂ is comprised in the second predetermined range of values R₂ and a dark color when the control value V₂ is comprised neither in the first nor in the second predetermined range of values R₁, R₂.

Thanks to the indicators, it is easy for an operator to visualize the gas measurement values V₁ having at least one control values V₂ affected with an indicator of the second type T₂ or of the third type T₃. It is therefore easy for the operator to determine the quality of the measurement of each gas measurement value V₁ and to determine the gas measurement value V₁ that might be erroneous.

Optionally, the evaluation 240 comprises an intervention of an operator during which the operator validates and/or modifies each indicator affected to each control value V₂ manually. In particular, an operator can choose to change the indicator affected to a gas measurement value V₁.

Thereafter, the method comprises filtering out (box 250) the gas measurement value V₁ when the indicator assigned to at least one of the matched control value V₂ is of the third type T₃ of indicator or indicates that the matched control value V₂ is not in a normal state. In other words, the gas measurement value V₁ is filtered out when at least one of the matched control value V2 is neither comprised in the first predetermined range of values R₁, nor in the second predetermined range of values R₂.

In another embodiment of the disclosure, the gas measurement value V₁ are filtered when the indicator assigned to at least one of the matched control value V₂ is of the second type T₂ or the third type T₃ of indicator. In other words, the gas measurement value V₁ is filtered out when at least one of the matched control value V₂ is not comprised in the first predetermined range of values R₁.

During the filtering 250, the gas measurement values V₁ to be filtered are deleted. The gas measurement values V₁ to be filtered can also be adjusted to neighboring gas measurement value V₁ through linear interpolation.

The filtering 250 is carried out by the calculation system 20 in interaction with the software application 140.

Then, the method comprises calculating (box 260) the depth at which each sample of the drilling fluid was extracted. Hence, a depth value is assigned to each gas measurement value V₁.

The calculation 260 comprises matching each measuring time t₁ with a depth calculated from internal characteristics of the drilling fluid installation 11. The internal characteristics are especially the depth of the drill bit 33, the flow rate of mud and the geometrical architecture of the drilling fluid installation 11.

The calculation260 is known from the prior art, in particular from US 2007/0062272 hereby incorporated by reference and will not be described in further detail.

Therefore, the method according to the disclosure is adapted for accurately determining erroneous gas measurement or artifacts during a mud logging analysis. The erroneous data and artifacts can then be removed or linearly interpolated.

The affectation of an indicator to a control value V₂ is automatic. The suspicious gas measurement values V₁ have at least one of their control values V2 affected with an indicator of the second type T₂ or of the third type T₃. An operator can therefore check more carefully the gas measurement values V₁ having at least one control value V₂ affected with an indicator of the second type T₂ or of the third type T₃. The operator has also the possibility to validate or modify each indicator.

The operator can also change the extreme values of the first predetermined range of values R₁ and of the second predetermined range of values R₂. The affectation of a type of indicator to a control value V₂ is therefore adjustable depending on the parameters fixed by the operator.

Moreover, the determination of erroneous gas measurement and artifacts is reproducible, that is to say independent on the operator. It is also quicker than a manual filtering as the intervention of the operator is not necessarily required.

In view of the entirety of the present disclosure, including the figures, a person skilled in the art should appreciate that they may readily use the present disclosure as a basis for designing or modifying other processes and structures for carrying out the same uses and/or achieving the same aspects introduced herein. A person skilled in the art should also realize that such equivalent constructions do not depart from the spirit and scope of the present disclosure, and that they may make various changes, substitutions and alterations herein without departing from the spirit and scope of the present disclosure. For example, although the preceding description has been described herein with reference to particular means, materials and embodiments, it is not intended to be limited to the particulars disclosed herein; rather, it extends to functionally equivalent structures, methods, and uses, such as are within the scope of the appended claims.

## Claims

1. A method for processing data collected during a mud logging analysis, comprising:
- providing (200), at a plurality of measuring times (t₁), a set of gas measurement values (V₁) representative of the quantity of at least one gas contained in several samples of a drilling fluid;
wherein the method comprises:
- providing (210), at a plurality of controlling times (t₂), a set of control values (V₂) of at least one control parameter relative to at least a part of an analysis unit (19) for providing the gas samples and/or the gas measurement values (V₁);
- matching (230), for each sample of the drilling fluid, the given gas measurement value (V₁) measured at a given measuring time (t₁) with at least one control value (V₂) measured at a controlling time (t₂) so that the control values (V₂) are representative of the part of the analysis unit (19), when the sample was processed in said part of the analysis unit (19);
- evaluating (240) whether each control value (V₂) of the at least one control parameter is in a normal state by comparing the control value (V₂) with at least a predetermined value, the predetermined value(s) being specific to the at least one control parameter, in order to assign, to each control value (V₂), an indicator of the quality of the measurement of each gas measurement value (V₁) matched with the control value (V₂).

2. A method according to claim 1, comprising filtering out (250) a gas measurement value (V₁) when at least one indicator assigned to one of the matched control value (V₂) indicates that the matched control value (V₂) is not in a normal state.

3. A method according to any of claims 1 or 2, comprising, after the filtering (250), calculating (260) the depth at which each sample of the drilling fluid was extracted in order to assign a depth to each gas measurement value (V₁) measured at a measuring time (t₁).

4. A method according to any one of the preceding claims, wherein the evaluation includes evaluating whether the control value (V₂) is comprised in a first predetermined range of values (R₁), the first predetermined range of values (R₁) being specific to the at least one control parameter.

5. A method according to any one of the preceding claims, wherein the evaluation (240) further comprises the determination whether each control value (V₂) of the at least one control parameter is comprised in a second predetermined range of values (R₂), the first predetermined range of values (R₁) being included in the second predetermined range of values (R₂), the indicator assigned to each control value (V₂) being of a first type (T₁) when the control value (V₂) is comprised in the first predetermined range of values (R₁), of a second type (T₂) when the control value (V₂) is comprised in the second predetermined range of values (R₂) and of a third type (T₃) when the control value (V₂) is included neither in the first nor in the second predetermined range of values (R₁, R₂).

6. A method according to any one of the preceding claims comprising:
- assessing (220), for each control parameter, a determined time difference (Δt) between, the measurement of a gas measurement value (V₁) of a sample of the drilling fluid and the measurement of a control value (V₂) of each control parameter associated to the same sample of the drilling fluid, by experiment or/and calculation, and
- the matching (230) comprising: adding to the controlling times (t₂) of the control values (V2) of each control parameter, the assessed time difference (Δt) corresponding to the control parameter, to obtain an adjusted time (tₐ) for each control value (V₂), and affecting each control value (V₂) to the gas measurement value (V₁) whose measuring time (t₁) is the closest to the adjusted time (tₐ) of the control value (V₂).

7. A method according to any one of the preceding claims, wherein the evaluation (240) comprises validating and /or modifying each indicator affected to each control value (V₂) manually.

8. A method according to any one of the preceding claims wherein the providing (210) of the gas measurement value (V₁) comprises:
- introducing a sample of a drilling fluid in an extractor (53) of the analysis unit (19);
- extracting a gas stream containing the gas to be measured out of the extractor (53); and
- introducing at least part of the gas stream in an analyzer (55) of the analysis unit(19) to obtain a gas measurement value (V₁).

9. A method according to claim 8, wherein the providing (220) of the control values (V₂) comprises measuring at least one control parameter chosen among the following group: a mud level in the extractor (53), a flow rate of mud introduced in the extractor (53), a temperature of the gas stream inside the extractor (53), a pressure of the gas stream after being extracted and filtered, before entering the analyzer (55), a flow rate of the gas stream in a pump (107) and a pressure of the gas stream in a pump (107).

10. A calculation system (20) comprising a processing unit (134) in interaction with a software application (140) for the implementation of the method according to any one of the preceding claims.

11. A mud logging installation (11), comprising:
- an analysis unit (19) for providing gas samples and/or gas measurement values representative of the quantity of at least one gas contained in several samples of a drilling fluid extracted from a wellbore, comprising :
• a sampling unit (51) for sampling the drilling fluid,
• an extractor (53) able to extract the gases contained in the samples of the drilling fluid,
• an analyzer (55) able to measure the quantity of at least one gas contained in the samples of the drilling fluid, and
at least one sensor (142, 144, 146, 148, 150, 152) able to measure at least one control parameter of the analysis unit (19) for providing gas samples and/or gas measurement values,
- a calculation system (20) according to claim 9.
